# EUROPEAN PATENT SPECIFICATION

(11) **EP 1 014 938 B1**
(45) Date of publication and mention of the grant of the patent: **31.07.2002**
(21) Application number: 98938851.7
(22) Date of filing: 28.08.1998
(51) Int. Cl.: A61K 7/50

(54) **PRE-MOISTENED WIPE**
ANGEFEUCHTETES WISCHTUCH
CHIFFON PRE-HUMIDIFIE

(30) Priority: 16.09.1997 US 931380
(43) Date of publication of application: 05.07.2000
(73) Proprietor: THE PROCTER & GAMBLE COMPANY, Cincinnati, Ohio 45202 (US)
(72) Inventor: RICHARDS, Marc, Fredric, Corinth, KY 41010 (US)
(74) Representative: Kohol, Sonia
(86) International application number: IB9801338
(87) International publication number: WO9913860

(56) References cited:
- EP-A- 0 566 340

## Description

### FIELD OF THE INVENTION

The present invention is related to disposable wiping articles, and more particularly to pre-moistened wipes.

### BACKGROUND OF THE INVENTION

Pre-moistened cleansing wipes are well known, and are referred to as wet wipes, towelletes, and the like. Pre-moistened wipes include a substrate, such as a nonwoven web, pre-moistened with an aqueous lotion. The lotion typically includes one or more surface active materials (surfactants). The lotion can also include, a preservative, and a fragrance ingredient (perfume or other scenting agent).

The surfactants in commercially available pre-moistened wipes aid in cleaning the skin. Additionally, the surfactants can prevent precipitation of one or more ingredients in the lotion. For instance, in the absence of a sufficient level of surfactant in typical pre-moistened wipe lotion formulations, it is possible that the preservative component and the scenting agent can interact, resulting in precipitation of the preservative component out of solution. Such precipitation must be avoided to maintain the function of the preservative and to prevent the lotion from becoming "cloudy."

On the other hand, if the lotion contains a high level of surfactant, the lotion may exhibit a high level of foaming in use. However, it has been found that such high foaming levels can be undesirable. In particular, it has been found that some users of pre-moistened wipes, such as pre-moistened baby wipes, perceive a high foaming level as potentially leading to irritation. Such a perception of possible irritation harshness is especially undesirable in a pre-moistened baby wipe. Further, high foaming levels can be undesirable for surface wiping, in that foam can result in streaking.

One commercially available pre-moistened wipe marketed by The Procter & Gamble Company as PAMPERS RASH CARE brand Baby Wipes has a lotion having a relatively low foaming level. These wipes have a lotion which includes dimethicone in polymeric beads, as disclosed in U.S. Patent 4,904,524. Such wipes, while having a low foaming level, can be relatively expensive to manufacture. Further, handling and processing of the lotion can be difficult due to the insolubility of dimethicone in water.

Commonly assigned U.S. Patent 5,648,083 issued July 15, 1997 discloses a wet wipe product comprising a substrate and an emulsion composition which includes water, silicone oil including dimethicone, and a polymeric emulsifier. While the product disclosed in U.S. Patent 5,648,083 represents an advancement in the art, it requires the use of an emulsion to prevent phase separation of the silicone component.

Accordingly, it is an object of the present invention to provide a pre-moistened wipe having a relatively low level of surfactant.

Another object of the present invention is to provide a pre-moistened wipe in which preservative and/or fragrance components do not phase separate from the wipe lotion.

Another object of the present invention is to provide a wipe having a silicone based surfactant which is water soluble.

### SUMMARY OF THE INVENTION

The present invention comprises a pre-moistened wipe. The wipe comprises a substrate moistened with an aqueous lotion comprising a water soluble silicone based component. In particular, the wipe comprises a nonwoven web of fibers moistened with a lotion comprising a silicone based sulfosuccinate. The silicone based sulfosuccinate provides gentle and effective cleansing without a high level of surfactant. Additionally, the silicone based sulfosuccinate can provide a solubilization function for preventing precipitation of oil soluble components, such as fragrance components, vitamin extracts, plant extracts, and essential oils.

In one embodiment, the lotion comprises a silicone copolyol sulfosuccinate selected from the group consisting of disodium dimethicone copolyol sulfosuccinate and diammonium dimethicone copolyol sulfosuccinate.

The lotion comprises less than about 1.00 percent by weight of the silicone based sulfosuccinate. In particular, the lotion can comprise less than about 0.20 percent by weight of the silicone based sulfosuccinate. Preferably, the lotion comprises between about 0.08 and about 0.10 percent by weight of the silicone based sulfosuccinate.

The lotion is an aqueous lotion, and preferably comprises at least about 85 percent by weight water, more preferably at least 90 percent by weight water, and still more preferably at least about 95 percent by weight water. Preferably, the lotion comprises no more than about 1.00 percent by weight total surfactant solids, including solids of the silicon based sulfosuccinate. The resulting lotion provides gentle yet effective cleaning.

The substrate can comprise an airlaid web of nonwoven fibers and a latex binder, and the substrate can have a dry basis weight of at least about 40 grams per square meter. The substrate can be wetted with an amount of lotion which is at least 100 percent of the dry weight of the substrate (at least one gram of lotion per gram of dry weight of the substrate).

### DETAILED DESCRIPTION OF THE INVENTION

The pre-moistened wipes of the present invention comprise a substrate moistened with a lotion. The substrate can comprise a woven or nonwoven web formed of natural fibers, synthetic fibers, or combinations thereof. The lotion comprises an aqueous lotion (at least 50 percent by weight water) which includes a water soluble silicon based surfactant.

The term "pre-moistened wipe" refers to a wipe which includes a substrate which is moistened, such as by wetting the substrate with a liquid composition, prior to use by the consumer. In particular, "pre-moistened wipe" refers to wipes having a substrate which is moistened prior to packaging, such as in a generally moisture impervious container or wrapper.

Such pre-moistened wipes, which can also be referred to as "wet wipes" and "towelettes", are suitable for use in cleaning male and female babies, as well as adults of all ages. Such wipes also include articles used for application of substances to the body, including but not limited to application of make-up, skin conditioners, ointments, and medications. Such wipes can also include such articles used for cleaning or grooming of pets, and articles used for general cleansing of surfaces and objects, such as household kitchen and bathroom surfaces, eyeglasses, exercise and athletic equipment, automotive surfaces, and the like.

As used herein, the term "weight percent" or "percent by weight" is meant to refer to the quantity by weight of a component in the lotion of the wipe as a percentage of the total weight of the lotion.

As used herein, the term "water soluble" means that a component is soluble or otherwise dispersable (such as to provide a micellar solution) in water at a level of at least about 0.25 percent by weight at 25 degrees Centigrade.

As used herein, the term "surfactant" refers to materials which preferably orient toward an interface, classes of surfactants including nonionic surfactants, anionic surfactants, cationic surfactants, amphoteric surfactants, zwitterionic surfactants, and mixtures thereof.

As used herein, the term "solubilizer" means a component that reduces the tendency of one or more other components in a lotion composition to phase separate from the lotion.

### SUBSTRATE:

Referring to the components of the present invention in more detail, the pre-moistened wipe of the present invention includes a substrate comprising a woven or nonwoven web of natural fibers, synthetic fibers, or mixtures of natural and synthetic fibers. Suitable natural fibers include but arc not limited to cellulosic fibers, such as wood pulp fibers, cotton, and rayon. Suitable synthetic fibers include fibers commonly used in textiles, including but not limited to polyester and polypropylene fibers.

Various forming methods can be used to form a suitable fibrous web for use in the present invention. For instance, the web can be made by nonwoven dry forming techniques, such as air-laying, or alternatively by wet laying, such as on a papermaking machine. Other nonwoven manufacturing techniques, including but not limited to techniques such as melt blown, spunbonded, needle punched, and hydroentanglement methods may also be used.

In one embodiment, the dry fibrous web can be an airlaid nonwoven web comprising a combination of natural fibers, staple length synthetic fibers and a latex binder. The dry fibrous web can be about 20-80 percent by weight wood pulp fibers, 10-60 percent by weight staple length polyester fibers, and about 10-25 percent by weight binder.

The dry, fibrous web can have a basis weight of between about 40 and about 80 grams per square meter. The density of the dry web can be measured after evaporating the liquid from the premoistened wipe, and the density can be less than about 0.12 grams per cubic centimeter. The density is the basis weight of the dry web divided by the thickness of the dry web, measured in consistent units, and the thickness of the dry web is measured using a circular load foot having an area of about 2 square inches and which provides a confining pressure of about 95 grams per square inch. In one embodiment, the dry web can have a basis weight of about 64 grams per square meter, a thickness of about 0.06 cm, and a density of about 0.11 grams per cubic centimeter.

In one embodiment, the dry fibrous web can comprise at least 50 percent by weight wood pulp fibers, and more preferably at least about 70 percent by weight wood pulp fibers. One particular airlaid nonwoven web which is suitable for use in the present invention comprises about 73.5 percent by weight cellulosic fibers (Southern softwood Kraft having an average fiber length of about 2.6 mm); about 10.5 percent by weight polyester fibers having a denier of about 1.35 gram/9000 meter of fiber length and a staple length of about 0.85 inch; and about 16 percent by weight of a binder composition comprising a styrene butadiene copolymer. The binder composition can be made using a latex adhesive commercially available as Rovene 5550 (49 percent solids styrene butadiene) available from Mallard Creek Polymers of Charlotte, N.C.

One suitable airlaid nonwoven web for use in the present invention is the airlaid nonwoven web employed in PAMPERS BABY FRESH brand baby wipes marketed by The Procter & Gamble Co. of Cincinnati, Ohio.

The pre-moistened wipe is made by wetting the dry substrate with at least 1 gram of liquid lotion per gram of dry fibrous web. Preferably, the dry substrate is wetted with at least about 2.0, and more preferably at least about 2.5 grams of liquid lotion per gram of the dry fibrous web.

The following patents are cited for their disclosure related to webs: U.S. Patent 3,862,472 issued Jan 28, 1975; U.S. Patent 3,982,302 issued Sept. 28, 1976; U.S. Patent 4,004,323 issued Jan. 25, 1977; U.S. Patent 4,057,669 issued Nov. 8, 1977; U.S. Patent 4,097,965 issued July 4, 1978; U.S. Patent 4,176,427 issued Dec. 4, 1979; U.S. Patent 4,130,915 issued Dec. 26, 1978; U.S. Patent 4,135,024 issued Jan. 16, 1979; U.S. Patent 4,189,896 issued Feb. 26, 1980; U.S. Patent 4,207,367 issued June 10, 1980; U.S. Patent 4,296,161 issued Oct. 20, 1981; U.S. Patent 4,309,469 issued Jan 25, 1982; U.S. Patent 4,682,942 issued July 28, 1987; and U.S. Patents 4,637,859; 5,223,096; 5.240,562; 5,556,509; and 5,580,423.

In one alternative embodiment, the substrate can comprise a hydroentangled web having a basis weight of about 62 grams per square meter and comprising about 50 percent by weight rayon fibers and about 50 percent by weight polyester fibers, polypropylene fibers, or a combination thereof. In another alternative embodiment, the substrate can comprise a laminate of two outer hydroentangled webs, such as nonwoven webs of polyester fibers having a basis weight of about 30 grams per square meter, joined to an inner constraining layer, which can be in the form of net-like scrim material which contracts upon heating to provide surface texture in the outer layers.

### LOTION:

The pre-moistened wipe of the present invention comprises an aqueous lotion. The lotion is preferably at least about 85 percent by weight water, more preferably at least about 90 percent by weight water, and still more preferably at least about 95 percent by weight water.

The lotion also comprises a water soluble silicon based surfactant. In one embodiment, the lotion comprises an anionic silicon based sulfosuccinate surfactant. Suitable counter ions include those derived from the alkai metals (e.g. sodium, potassium); the alkaline earth metals (e.g. magnesium, calcium); ammonia, and alkanol amines (e.g. mono, di, and tri ethanol amines).

In one embodiment of the present invention, the lotion comprises a silicon copolyol sulfosuccinate selected from the group consisting of disodium dimethicone copolyol sulfosuccinate and diammonium dimethicone copolyol sulfosuccinate.

The lotion preferably comprises less than about 1.00 percent by weight of the silicone based sulfosuccinale. In particular, the lotion can comprise less than about 0.20 percent by weight of the silicone based sulfosuccinate, and in embodiment comprises between about 0.08 and about 0.10 percent by weight of the silicone based sulfosuccinate. Preferably, the lotion comprises no more than about 1.00 percent by weight total surfactant solids, including the silicone based sulfosuccinate.

A suitable disodium dimethicone copolyol sulfosuccinate is commercially available as MACKANATE DC-30 and MACKANATE DC-50 brand sulfosuccinate surfactants available from the Mclntyre Group, LTD, University Park, Illinois. A suitable diammonium dimethicone copolyol sulfosuccinate is commercially available as MACKANATE DC-30A from the same supplier. U.S. Patent 4,849,127 issued July 18, 1989 to Maxon is cited for its disclosure related to dimethicone copolyol sulfosuccinates.

The lotion preferably also comprises one or more of the following: an effective amount of a preservative, an effective amount of a humectant, an effective amount of an emollient; an effective amount of a fragrance, and an effective amount of a fragrance solubilizer.

As used herein, an emollient is a material that softens, soothes, supples, coats, lubricates, or moisturizes the skin. The term emollient includes, but is not limited to, conventional lipid materials (e.g. fats, waxes), polar lipids (lipids that have been hydrophylically modified to render them more water soluble), silicones, hydrocarbons, and other solvent materials. Emollients useful in the present invention can be petroleum based, fatty acid ester type, alkyl ethoxylate type, fatty acid ester ethoxylates, fatty, alcohol type, polysiloxane type, mucopolysaccharides, or mixtures thereof.

Humectants are hygroscopic materials that function to draw water into the stratum corneum to hydrate the skin. The water may come from the dermis or from the atmosphere. Examples of humectants include glycerin, propylene glycol, and phospholipids.

Fragrance components, such as perfumes, include, but are not limited to water insoluble oils, including essential oils.

Fragrance solubilizers are components which reduce the tendency of the water insoluble fragrance component to precipitate from the lotion. Examples of fragrance solubilizers include alcohols such as ethanol, isopropanol, benzyl alcohol, and phenoxyethanol; any high HLB (HLB greater than 13) emulsifier, including but not limited to polysorbate; and highly ethoxylated acids and alcohols.

Preservatives prevent the growth of micro-organisms in the liquid lotion and/or the substrate. Generally, such preservatives are hydrophobic or hydrophillic organic molecules. Suitable preservatives include, but are not limited to parabens, such as methyl parabens, propyl parabens, and combinations thereof.

The lotion can also comprise an effective amount of a kerotolytic for providing the function of encouraging healing of the skin. An especially preferred kerotolytic is Allantoin ((2,5-Dioxo-4-Imidazolidinyl)Urca), a helerocyclic organic compound having an empirical formula C₄H₆N₄O₃. Allantoin is commercially available from Tri-K Industries of Emerson, New Jersey. It is well recognized that the long term wear of disposable absorbent structures, such as disposable diapers, may lead to skin which is compromised in terms of being over hydrated. It is generally known that hyperhydrated skin is more susceptible to skin disorders, including heat rash, abrasion, pressure marks and skin barrier loss. For example, 21 CFR 333.503 defines diaper rash as an inflammatory skin condition in the diaper area (perineum, buttocks, lower abdomen, and inner thighs) caused by one or more of the following factors: moisture, occlusion, chafing, continued contact with urine or feces, or mechanical or chemical irritation. A premoistened wipe according to the present invention can include an effective amount of allantoin for encouraging the healing of skin, such as skin which is over hydrated.

U.S. Patent 5,534,265 issued July 9, 1996; U.S. Patent 5,043,155 issued August 27, 1991; and U.S. Patent 5,648,083 issued July 15, 1997 are cited for the purpose of disclosing additional lotion ingredients.

A suitable lotion comprises at least 95 percent by weight water, between about 0.08 to about 0.10 percent by weight disodium dimethicone copolyol sulfosuccinate, which can serve as a surfactant; between about 0.5 to about 5 percent by weight propylene glycol, which can serve as a humectant; between about 0.1 to about 1 percent by weight Polysorbate 20, which can serve as an emulsifier for solubilizing fragrance components; about 0.01 to about 0.5 percent by weight Methylparaben, which can serve as a preservative; about 0.01 to about 0.1 percent by weight Propylparaben, which can serve as a preservative; about 0.01 to about 0.1 percent by weight 2-Bromo-2-Nitropropane-1, 3-Diol which can serve as a preservative; and about 0.01 to about 1 percent by weight of one or more fragrance components.

The lotion can further comprisc between about 0.1 and about 3 percent by weight Allantoin, and about 0.1 to about 10 percent by weight of an aloe extract, such as aloe vera, which can serve as an emollient. Aloe vera extract is available in the form of a concentrated powder from the Rita Corporation of Woodstock, Ill..

The silicone based sulfosuccinate provides effective yet gentle cleaning. It is also believed that the silicone based sulfosuccinate provides secondary solubilization of the fragrance oils in the aqueous lotion.

Without being limited by theory, it is believed that in typical wipe lotions, the oil soluble materials, such as fragrances, can interact with marginally soluble aromatic compounds (preservatives such as methyl and propyl parabens) causing the aromatic compounds to precipitate out of solution. As a result, the lotion can be unacceptable for use in a consumer product because of inadequate preservation of the lotion.

Typically, fragrance solubilizer levels are increased to compensate for this problem, or additional solubilizers are required. However, without being limited by theory, it is believed that increasing the solubilizer level can, in turn, weaken the preservative system. For example, it is believed that a solubilizer, such as polysorbate, can cause paraben partitioning into the oil phase of personal use products. As a result, these parabens are not available to serve their function as preservatives.

The present invention avoids these problems in that the silicon based sulfosuccinate provides: a lotion with lower total surfactant solids and less irritation potential, as well as secondary solubilization of the fragrance component, to reduce phase separation of the fragrance component and to preserve the function of the preservatives.

### Example 1:

A suitable lotion comprising a silicone based sulfosuccinate can be made according to the following example to provide a 500 gram batch:
A first premix (premix A) is prepared by mixing together:
7.50 grams of propylene glycol (liquid), which serves as a solvent/humectant;
1.15 grams of Methyl/Propyl Paraben (powder), which serves as preservative.

A second premix (premix B) is prepared by mixing together:
1.00 grams of Polysorbate 20 (liquid), which serves as a fragrance solubilizer;
0.19 grams of fragrance oil (liquid).
Premix B is then added to 250 grams of deionized water, with agitation.
0.25 grams of Bronopol is then added to this mixture. The Bronopol (2-bromo-2-nitropropane-1,3-diol) (powder) serves as a preservative.
1.5 grams of Mackanate DC 30 liquid (30 percent by weight disodium dimethicone copolyol sulfosuccinate solids) is then added to this mixture.
2.5 grams of Allantoin solids (powder) is then added to this mixture.
Sufficient deionized water is then added to this mixture to q.s. to 100 percent.
Premix A is then added to this mixture, with agitation.

The lotion made according to the above example comprises about 0.09 percent by weight disodium dimethicone copolyol sulfosuccinate, and about 0.5 percent by weight Allantoin.

### Example 2:

A lotion according to this example is made as described above for Example 1, with the following modification. Prior to adding sufficient deionized water to q.s. to 100 percent, 0.0125 grams of a concentrated aloe vera extract powder (200 to 1 concentration) is added to the mixture. The resulting lotion comprises about 0.5 percent by weight aloe vera extract.

## Claims

1. A disposable wiping article, the disposable wiping article comprising a substrate pre-moistened with a lotion, and wherein the lotion comprises a silicone copolyol sulfosuccinate.

2. The wiping article of Claim 1 wherein the lotion comprises a silicone copolyol sulfosuccinate selected from the group consisting of disodium dimethicone copolyol sulfosuccinate and diammonium dimethicone copolyol sulfosuccinate.

3. The wiping article of Claims 1 or 2 wherein lotion comprises less than 1.00 percent, preferably less than 0.20 percent, and more preferably between 0.08 and 0.10 percent by weight of the silicone copolyol sulfosuccinate.

4. The wiping article of Claims 1, 2, or 3 wherein the lotion comprises at least 85 percent and more preferably at least 90 percent by weight water.

5. The wiping article of Claims 1, 2, 3, or 4 wherein the substrate is wetted with at least 1.0 gram, preferably at least 2.0 grams of lotion per gram of dry substrate weight.

6. The wiping article of Claims 1, 2, 3, 4, 5 further comprising an effective amount of a preservative.

7. The wiping article of Claims 1, 2, 3, 4, 5, or 6 further comprising an effective amount of a fragrance and an effective amount of a fragrance solubilizer.

8. The wiping article of Claims 1, 2, 3, 4, 5, 6, or 7 further comprising a kerotolytic, and wherein the kerotolytic preferably comprises allantoin.

## Patentansprüche

1. Wegwerfbarer Wischartikel, wobei der wegwerfbare Wischartikel aufweist ein mit einer Lotion vorbefeuchtetes Substrat und in welchem die Lotion ein Siliconcopoliolschwefelsuccinat umfaßt.

2. Wischartikel nach Anspruch 1, in welchem die Lotion ein Siliconcopoliolschwefelsuccinat ausgewählt aus der Gruppe bestehend aus Dinatriumdimethiconcopoliolschwefelsuccinat und Diammoniumdimethiconcopoliolschwefelsuccinat umfaßt.

3. Wischartikel nach den Ansprüchen 1 oder 2, in welchem die Lotion weniger als 1,00 Gew.%, vorzugsweise weniger als 0,20 Gew.% und ganz bevorzugt zwischen 0,08 und 0,10 Gew.% Siliconcopoliolschwefelsuccinat aufweist.

4. Wischartikel nach den Ansprüchen 1, 2 oder 3, in welchem die Lotion wenigstens 85 Gew.% und vorzugsweise wenigstens 90 Gew.% Wasser aufweist.

5. Wischartikel nach den Ansprüchen 1, 2, 3 oder 4, in welchem das Substrat mit wenigstens 1,0 Gramm, vorzugsweise wenigstens 2,0 Gramm Lotion pro Gramm Trockensubstratgewicht benetzt ist.

6. Wischartikel nach den Ansprüchen 1, 2, 3, 4, 5, ferner mit einer effektiven Menge eines Konservierungsmittels.

7. Wischartikel nach den Ansprüchen 1, 2, 3, 4, 5 oder 6, ferner mit einer effektiven Menge eines Duftstoffes und einer effektiven Menge eines Duftstoffauslösers.

8. Wischartikel nach den Ansprüchen 1, 2, 3, 4, 5, 6 oder 7, ferner mit einem Kerotolytmittel und in welchem das Kerotolytmittel vorzugsweise Allantoin aufweist.

## Revendications

1. Article d'essuyage à jeter après usage, l'article d'essuyage à jeter après usage comprenant un substrat préhumidifié avec une lotion, et dans lequel la lotion comprend un silicone-copolyol-sulfosuccinate.

2. Article d'essuyage selon la revendication 1 dans lequel la lotion comprend un silicone-copolyol-sulfosuccinate choisi dans le groupe constitué par le diméthicone-copolyol-sulfosuccinate de disodium et le diméthicone-copolyol-sulfosuccinate de diammonium.

3. Article d'essuyage selon la revendication 1 ou 2 dans lequel la lotion comprend moins de 1,00 pour cent, de préférence moins de 0,20 pour cent et mieux encore entre 0,08 et 0,10 pour cent en poids du silicone-copolyol-sulfosuccinate.

4. Article d'essuyage selon la revendication 1, 2 ou 3 dans lequel la lotion comprend au moins 85 pour cent et mieux encore au moins 90 pour cent en poids d'eau.

5. Article d'essuyage selon la revendication 1, 2, 3 ou 4 dans lequel le substrat est mouillé avec au moins 1,0 gramme, de préférence au moins 2,0 grammes, de lotion par gramme de poids de substrat sec.

6. Article d'essuyage selon la revendication 1, 2, 3, 4 ou 5 comprenant en outre une quantité efficace d'un conservateur.

7. Article d'essuyage selon la revendication 1, 2, 3, 4, 5 ou 6 comprenant en outre une quantité efficace d'un parfum et une quantité efficace d'un solubilisant pour parfum.

8. Article d'essuyage selon la revendication 1, 2, 3, 4, 5, 6 ou 7 comprenant en outre un cérotolytique, et dans lequel le cérotolytique comprend de préférence l'allantoïne.
